(19) **Europäisches Patentamt / European Patent Office / Office européen des brevets**

(11) **EP 2 116 234 A1**

(12) **EUROPEAN PATENT APPLICATION**
published in accordance with Art. 153(4) EPC

(43) Date of publication:
**11.11.2009 Bulletin 2009/46**

(51) Int Cl.:
**A61K 9/70** (2006.01)  **A61K 31/137** (2006.01)
**A61K 31/192** (2006.01)  **A61K 31/195** (2006.01)
**A61K 31/405** (2006.01)  **A61K 47/10** (2006.01)
**A61K 47/12** (2006.01)  **A61K 47/22** (2006.01)
**A61K 47/32** (2006.01)  **A61K 47/44** (2006.01)

(21) Application number: **07850225.9**

(22) Date of filing: **06.12.2007**

(86) International application number:
**PCT/JP2007/073616**

(87) International publication number:
**WO 2008/069283 (12.06.2008 Gazette 2008/24)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **06.12.2006 JP 2006330005**

(71) Applicant: **NIPRO Patch Co., Ltd.
Saitama 344-0057 (JP)**

(72) Inventors:
• **KAWAMURA, Naohisa
Kasukabe-shi
Saitama 344-0057 (JP)**

• **SAITOH, Takashi
Kasukabe-shi
Saitama 344-0057 (JP)**
• **TSUCHIYA, Junko
Kasukabe-shi
Saitama 344-0057 (JP)**
• **SUGAYA, Chie
Kasukabe-shi
Saitama 344-0057 (JP)**

(74) Representative: **Maggs, Michael Norman et al
Kilburn & Strode LLP
20 Red Lion Street
London WC1R 4PJ (GB)**

(54) **PHARMACEUTICAL COMPOSITION FOR EXTERNAL APPLICATION AND ADHESIVE SKIN PATCH**

(57) Disclosed are a pharmaceutical composition for external application, an adhesive skin patch and others which are improved in a drug release property, transdermal absorbability and stability of a pharmacologically active ingredient contained therein and can reduce the aggravation of irritation to the skin. Specifically disclosed is an adhesive skin patch comprising a support and an adhesive layer laminated on the support, wherein the adhesive layer contains a pharmaceutical composition for external application. The pharmaceutical composition for external application comprises a pharmacologically active ingredient, an auxiliary component and a pyrrolidone compound, wherein one of the pharmacologically active ingredient and the auxiliary component has a carboxylate group in the molecule and the other has a hydroxy group in the molecule. Preferably, the adhesive layer further comprises a hydrogenated oil.

FIG. 1

EP 2 116 234 A1

**Description**

TECHNICAL FIELD

The present invention relates to a pharmaceutical composition for external application and a skin patch, and more particularly, relates to a pharmaceutical composition for external application and skin patch containing a drug.

BACKGROUND ART

Conventionally, transdermally absorptive skin patches containing a drug including a compound having a carboxylic acid group (for example, loxoprofen sodium, or diclofenac sodium) have been extensively known. For example, a skin patch containing loxoprofen sodium, a styrene-isoprene-styrene block copolymer and crotamiton in an adhesive layer (see Patent Document 1), a preparation for external application containing loxoprofen sodium and crotamiton (see Patent Document 2), and the like have been disclosed. In addition, a skin patch prepared by adding an organic acid to a nonsteroidal antiphlogistic analgetics (maleic acid or the like) (see Patent Document 3), and a transdermally absorbed preparation added with an inorganic acid (see Patent Document 4) have been disclosed.
Patent Document 1: Japanese Unexamined Patent Application, First Publication No. 2004-43512.
Patent Document 2: Japanese Unexamined Patent Application, First Publication No. Hei 10-120560.
Patent Document 3: Japanese Examined Patent Application, Second Publication No. Hei 7-47535.
Patent Document 4: International Publication Pamphlet No. WO2006/048939.

DISCLOSURE OF THE INVENTION

Problems to be Solved by the Invention

However, in the skin patch disclosed in Patent Document 1 or 2, solubility of the drug is inferior, and a problem with respect to skin irritation may be caused. Further, the drug solubility is not sufficiently improved according to the skin patch disclosed in Patent Document 3 or 4, and releaseability and transdermal absorptivity of the drug are also not satisfactory.
Moreover, when a compound having a hydroxyl group (for example, menthol) is included in the adhesive layer, the hydroxyl group of this compound may cause an esterification reaction with the carboxylic acid group carried by the drug. To the contrary, when a compound having a carboxylic acid group is included in the adhesive layer, the carboxylic acid group of this compound may cause an esterification reaction with the hydroxyl group carried by the drug. Thus, stability of the drug could not have been sufficiently achieved.
The present invention was made in view of the foregoing problems, and an object of the invention is to provide a pharmaceutical composition for external application and a skin patch capable of improving releaseability, transdermal absorptivity and stability of a drug, and which is also capable of suppressing enhancement of skin irritating properties.

Means for Solving the Problems

The present inventors found that the foregoing problems can be solved by blending a pyrrolidone compound in a pharmaceutical composition for external application, or in an adhesive layer of a skin patch. Accordingly, the present invention was accomplished. More specifically, the present invention provides the following aspects.

(1) According to a first aspect, a pharmaceutical composition for external application which is to be applied to the skin is provided,
the composition including a drug, an auxiliary ingredient, and a pyrrolidone compound, in which
one of the drug and the auxiliary ingredient includes a compound including a carboxylic acid group, and an other of the drug and the auxiliary ingredient includes a compound including a hydroxyl group.
(2) In a second aspect, the composition according to the first aspect is provided, in which the pyrrolidone compound is at least one selected from the group consisting of N-methyl-2-pyrrolidone, 2-pyrrolidone, 5-methyl-2-pyrrolidone, 1,5-dimethyl-2-pyrrolidone, 1-ethyl-2-pyrrolidone, 2-pyrrolidone-5-carboxylic acid, N-(2-hydroxyethyl)pyrrolidone, N-octyl-2-pyrrolidone, polyvinylpyrrolidone, N-vinyl-2-pyrrolidone, and methylvinylpyrrolidone.
(3) In a third aspect, the composition according to the second aspect is provided, in which the pyrrolidone compound is N-methyl-2-pyrrolidone.
(4) In a fourth aspect, the composition according to any one of the first to the third aspects is provided, further including a hydrogenated oil.
(5) In a fifth aspect, the composition according to any one of the first to the fourth aspects is provided, in which the compound included in the drug is at least one selected from the group consisting of loxoprofen, diclofenac, felbinac, ketoprofen, indometacin, tulobuterol, and a salt thereof.

(6) In a sixth aspect, the composition according to any one of the first to the fifth aspects is provided, in which the compound included in the auxiliary ingredient is at least one selected from the group consisting of a hydroxy acid, a menthols, and a polyhydric alcohol.

(7) In a seventh aspect, the composition according to the sixth aspect is provided, in which the hydroxy acid is at least one selected from the group consisting of lactic acid, tartaric acid, and citric acid.

(8) According to an eighth aspect, a skin patch is provided having a backing, and an adhesive layer laminated on the backing, in which

the adhesive layer contains the pharmaceutical composition for external application according to any one of the first to the seventh aspects.

(9) In a ninth aspect, the skin patch according to the eighth aspect is provided, in which the pyrrolidone compound is included in an amount of no less than 0.01% by mass and no greater than 20% by mass based on the mass of the entire adhesive layer.

(10) In a tenth aspect, the skin patch according to the eighth or ninth aspect is provided, in which the adhesive layer contains a styrene-isoprene-styrene block copolymer.

(11) According to an eleventh aspect, a skin patch is provided having a backing, and an adhesive layer laminated on the backing, in which

the adhesive layer contains a styrene-isoprene-styrene block copolymer, a tackifier, a plasticizer, diclofenac sodium, 1-menthol, N-methyl-2-pyrrolidone, a hydrogenated oil, and at least one of citric acid and lactic acid.

(12) According to a twelfth aspect, a method for suppressing esterification of respective compounds included in a drug and an auxiliary ingredient included in a pharmaceutical composition for external application to skin, is provided, in which one of the drug and the auxiliary ingredient comprises a compound including a carboxylic acid group, and an other of the drug and the auxiliary ingredient comprises a compound including a hydroxyl group.

(13) According to a thirteenth aspect, an esterification suppressing agent for use in a pharmaceutical composition for external application which is to be applied to the skin, is provided, in which the composition includes a drug and an auxiliary ingredient, in which one of the drug and the auxiliary ingredient includes a compound including a carboxylic acid group, and an other of the drug and the auxiliary ingredient includes a compound including a hydroxyl group, and in which

the agent includes a pyrrolidone compound in an effective amount for suppressing esterification of the drug and the auxiliary ingredient.

Effects of the Invention

According to the present invention, a drug and an auxiliary ingredient, one thereof including a compound having a carboxylic acid group and the other thereof including a compound having a hydroxyl group, are included in combination with a pyrrolidone compound; therefore, releaseability, transdermal absorptivity, and stability of the drug can be improved, and enhancement of skin irritating properties can be suppressed.

BRIEF DESCRIPTION OF THE DRAWINGS

Fig. 1 shows a graph illustrating stability of a drug including a compound having a carboxylic acid group;
Fig. 2 shows a graph illustrating stability of a drug including a compound having a carboxylic acid group;
Fig. 3 shows a graph illustrating stability of a drug including a compound having a carboxylic acid group;
Fig. 4 shows a graph illustrating stability of other drug including a compound having a carboxylic acid group;
Fig. 5 shows a graph illustrating stability of other drug including a compound having a carboxylic acid group; and
Fig. 6 shows a graph illustrating stability of a drug including a compound having a hydroxyl group.

PREFERRED MODE FOR CARRYING OUT THE INVENTION

One example of embodiments of the present invention will be explained below; however, the present invention is not limited to the following embodiment.

The pharmaceutical composition for external application of the present invention can be used in any applications as long as it is applied to the skin. More specifically, the composition may be directly applied to the skin, and may be in any form such as liquid (liquid formulation), cream (cream formulation) and gel (gel formulation), but is preferably blended in an adhesive layer of a skin patch applied to the skin (cataplasm, skin patch, transdermal absorptive preparation, plaster, tape, and the like).

Hereinafter, the skin patch is explained. The skin patch of the present invention has a backing, and an adhesive layer laminated on the backing.

Adhesive Layer

The adhesive layer that constitutes the skin patch of the present invention contains a pharmaceutical composition for external application. This pharmaceutical composition for external application at least contains a drug and an auxiliary ingredient, one thereof including a compound having a carboxylic acid group and the other thereof including a compound having a hydroxyl group, and a pyrrolidone compound.

In addition, the adhesive layer according to this embodiment may further contain a hydrogenated oil, a base, a tackifier, a plasticizer, and the like.

Drug

The drug includes a compound having a carboxylic acid group or a hydroxyl group. The compound which is included in the drug and has a carboxylic acid group is not particularly limited, and can include a commercially available medicinal agent or the like. Examples of such a drug include indometacin, ketoprofen, flurbiprofen, loxoprofen, ketorolac, felbinac, diclofenac, salicylic acid, glycol salicylate, acetyl salicylate, flufenamic acid, mefenamic acid, acemetacin, alclofenac, ibuprofen, sulindac, tolmetin, lobenzarit, penicillamine, oxaprozin, diflunisal, fenbufen, fentiazac, naproxen, pranoprofen, tiaprofen, suprofen, oxaprozin, etodolac, zaltoprofen, and salts thereof. Among these, loxoprofen, diclofenac, felbinac, ketoprofen, indometacin, or a salt thereof may be preferably used.

The compound which is included in the drug and has a hydroxyl group is not particularly limited, and can include a commercially available medicinal agent or the like. Examples of such a drug include tulobuterol, estradiol, ethynylestradiol, norethisterone, isosorbide mononitrate, scopolamine, buprenorphine, glycol salicylate, calcitriol, piroxicam meloxicam and salts thereof, and tulobuterol is more preferable.

Auxiliary Ingredient

The auxiliary ingredient includes a compound having a hydroxyl group when the drug includes a compound having a carboxylic acid, and the auxiliary ingredient includes a compound having a carboxylic acid group when the drug includes a compound having a hydroxyl group. Although the compound which is included in the auxiliary ingredient and has a hydroxyl group is not particularly limited, it may be exemplified by hydroxy acids, menthols, polyhydric alcohols, polyhydric alcohol fatty acid esters, and polyoxyethylene sorbitan fatty acid esters; and hydroxy acids, menthols and polyhydric alcohols are preferable.

The compound which is included in the auxiliary ingredient and has a carboxylic acid group is not particularly limited, and a variety of organic acids can be used. Specific examples include aliphatic (mono-, di- or tri-) carboxylic acids (acetic acid, propionic acid, citric acid (including citrate anhydride), isobutyric acid, caproic acid, caprylic acid, lactic acid, maleic acid, pyruvic acid, oxalic acid, succinic acid, tartaric acid, etc.), aromatic carboxylic acids (phthalic acid, salicylic acid, benzoic acid, acetyl salicylic acid, etc.), alkylsulfonic acids (methanesulfonic acid, ethanesulfonic acid, propylsulfonic acid, butanesulfonic acid, polyoxyethylene alkylether sulfonic acid, etc.), alkylsulfonic acid derivatives (N-2-hydroxyethylpiperidine-N'-2-ethanesulfonic acid, cholic acid derivatives (dehydrocholic acid, etc.), and salts of the same (for example, alkali metal salts such as sodium salts), and the like. Among these compounds, the carboxylic acids and salts thereof are preferred, and acetic acid and sodium acetate are particularly preferred. Such compounds may be used alone, or two or more thereof may be used in combination. Thus, the auxiliary ingredient refers to any ingredients except for the drug among the ingredients included in the adhesive layer.

The hydroxy acids are exemplified by glycolic acid, lactic acid, malic acid, tartaric acid, gluconic acid, salicylic acid, $\alpha$-oxybutyric acid, glyceric acid, citric acid and the like. Lactic acid, tartaric acid, and citric acid are preferred, and lactic acid is more preferred.

The menthols are preferably 1-menthol, mentha oil and the like, and 1-menthol is more preferred.

The polyhydric alcohols are exemplified by glycerin, and the like. The polyhydric alcohol fatty acid esters are exemplified by glyceryl monostearate, glyceryl dioleate and the like. In addition, polyoxyethylene sorbitan fatty acid esters are exemplified by monooleic acid polyoxyethylene sorbitan (for example, "polysorbate 80"), and the like.

When lactic acid is included as the auxiliary ingredient, the lactic acid which may be used is not particularly limited, and can be any one commonly used for medical drugs. The lactic acid may be either lactic anhydride, or a mixture of lactic acid and lactic anhydride. The optical activity is also not limited, and any one of racemic bodies and each isomer may be used. The content of the lactic acid is preferably no less than 0.05% by mass and no greater than 20% by mass, and more preferably no less than 0.1% by mass and more preferably no greater than 5% by mass based on the mass of the entire adhesive layer. A content that is too low of the lactic acid leads to failure in achieving sufficient improvement of releaseability and transdermal absorptivity of the drug, whereas a content that is too high is not economical. Pyrrolidone Compound

Conventionally, compositions including crotamiton, an organic acid, an inorganic acid or the like alone have been proposed, but the drug solubility has not been satisfactorily improved, and thus releaseability of the drug from the adhesive layer has not been enough. However, it was revealed, unexpectedly, that the solubility of the drugs is significantly improved by including a pyrrolidone compound in the adhesive layer, whereby diffusibility of the drug in an adhesive layer is improved, resulting in favorable releaseability from the adhesive layer, and transdermal absorptivity of the drug. In addition, it was proven that stability of the drug can be improved since esterification of respective compounds included in the drug and the auxiliary ingredient is suppressed. In other words, according to this embodiment, a skin patch having prominently superior releaseability, transdermal absorptivity and stability of drugs can be obtained, being far superior to that obtained by mere use of each ingredient alone.

The pyrrolidone compound for use in the present invention is not particularly limited, and a commonly used compound for medical drugs may be employed. Specific examples of the pyrrolidone compound include N-methyl-2-pyrrolidone, 2-pyrrolidone, 5-methyl-2-pyrrolidone, 1,5-dimethyl-2-pyrrolidone, 1-ethyl-2-pyrrolidone, 2-pyrrolidone-5-carboxylic acid, N-(2-hydroxyethyl)pyrrolidone, N-octyl-2-pyrrolidone, polyvinylpyrrolidone, N-vinyl-2-pyrrolidone, methylvinylpyrrolidone and the like, and N-methyl-2-pyrrolidone is preferred since the releaseability, transdermal absorptivity and stability of the drug can be further improved.

Since a content of the pyrrolidone compound that is too low or too high may lead to failure in sufficiently improving the releaseability, transdermal absorptivity or stability of the drug, the content is no less than 0.01% by mass and no greater than 20% by mass, and preferably no less than 0.1% by mass and no greater than 10% by mass based on the mass of the entire adhesive layer.

Furthermore, the pyrrolidone compound is blended in an amount of preferably no less than 1 part by mass and no greater than 1000 parts by mass, and more preferably no less than 2 parts by mass and no greater than 200 parts by mass with respect to 100 parts by mass of the lactic acid. A too low or too high content ratio of the pyrrolidone compound to the lactic acid may lead to failure in sufficiently improving the drug stability, drug solubility, or drug transdermal absorptivity. Hydrogenated Oil

The hydrogenated oil is a kind of fat and oil produced by adding hydrogen to the double bond of a liquid unsaturated fatty acid such as fish oil, thereby raising the melting point to permit hardening. The hydrogenated oil used in the present invention, which is not particularly limited, may be a hydrogenated product of a material such as cottonseed oil, soybean oil, castor oil, rapeseed oil, palm oil, fish oil or the like. Hydrogenated oils are more preferred than partially hydrogenated oils, and specifically, hydrogenated castor oil is exemplified. In addition, the melting point may be approximately 80 to 90°C, while the content may be no less than 0.1% by mass based on the mass of the entire adhesive layer.

The content of the hydrogenated oil is preferably no less than 0.5% by mass, and more preferably no less than 1% by mass based on the mass of the entire adhesive layer since the releaseability and stability of the drug can be sufficiently improved. The upper limit of the content, although not limited in particular, may be no greater than 20% by mass, and is preferably no greater than 5% by mass based on the mass of the entire adhesive layer. The range of the content of the hydrogenated oil is most preferably no less than 0.5% by mass and no greater than 5% by mass based on the mass of the entire adhesive layer.

Base

The base for use in the present invention is exemplified by styrene-isoprene-styrene block copolymers (hereinafter, may be also referred to as "SIS copolymer"). The SIS copolymer is a kind of rubber-based adhesives, which is a member of A-B-A type polymers, and is a styrene-based thermoplastic elastomer having a molecular structural model in which the end block "A" is polystyrene, while the rubber middle block "B" is polyisoprene. The styrene-isoprene-styrene block copolymer for use in the present invention is not particularly limited, and a conventionally used one may be employed. In general, the copolymer having a mass ratio of styrene to isoprene (styrene/isoprene) being 10/90 to 30/70 and preferably 20/80 to 25/75, and having a solution viscosity (MPa · [cps] at 25°C) of about 100 to 3,000 may be used.

Specific examples include commercially available products such as one having a mass ratio (styrene/isoprene) of styrene to isoprene being 15/85 and having a solution viscosity (MPa [cps] at 25°C) of 1,500 (trade name: Kraton D-1107), one having styrene/isoprene of 15/85 and having a solution viscosity (MPa · [cps] at 25°C) of 900 (trade name: Kraton D-1112), one having styrene/isoprene of 17/83 and having a solution viscosity (MPa · [cps] at 25°C) of 500 (trade name: Kraton D-1117P), one having styrene/isoprene of 22/78 (trade name: Kraton D-KX401), one having styrene/isoprene of 16/84 (trade name: Kraton D-KX406), one having styrene/isoprene of 30/70 and having a solution viscosity (MPa · [cps] at 25°C) of 300 (trade name: Kraton D-1125x), one having styrene/isoprene of 10/90 and having a solution viscosity (MPa · [cps] at 25°C) of 2,500 (trade name: Kraton D-1320x), and the like (all available from Kraton JSR Elastomers K.K.), which may be used either alone, or in combination of two or more thereof.

In the present invention, although not particularly limited, one having a high styrene mass proportion is preferably used, and specifically, one having a styrene/rubber ratio (% by mass) of 22/78 (trade name: Kraton D-KX401) may be included. The content of the SIS copolymer used in the present invention, although not limited in particular, is preferably 10 to 40% by mass based on the mass of the entire adhesive layer.

Tackifier

The tackifier which may be suitably used in the present invention, although not particularly limited, is an alicyclic saturated hydrocarbon resin (synthetic petroleum resin), a rosin ester derivative, a terpene-based resin, a phenol-based resin or the like, which may be used alone or in combination of two or more thereof. The tackifier, although not limited in particular, is preferably included in an amount of 10 to 35% by mass based on the mass of the entire adhesive layer.
The alicyclic saturated hydrocarbon resin is exemplified by ARKON P-100 (trade name: manufactured by Arakawa Chemical Industries, Ltd.). The rosin ester derivative is exemplified by Ester gum H (trade name: manufactured by Arakawa Chemical Industries, Ltd.), KE-311 (trade name: manufactured by Arakawa Chemical Industries, Ltd.) and KE-100 (trade name: manufactured by Arakawa Chemical Industries, Ltd.). The terpene-based resin is exemplified by YS resin (trade name: manufactured by YASUHARA CHEMICAL Co., Ltd.).

Plasticizer

The plasticizer for use in the present invention is not particularly limited, and generally used one may be employed. Specific examples include liquid paraffin, higher alcohols such as octyldodecanol, squalane, squalene, castor oil, liquid rubbers (polybutene), and fatty acid esters such as isopropyl myristate and isopropyl palmitate. Of these, liquid paraffin is preferred. The plasticizer, although not limited in particular, may be included generally in an amount of 20 to 60% by mass based on the mass of the entire adhesive layer. Optional Ingredient
The skin patch of the present invention may also include an excipient, an anti-oxidizing agent, a solubilizer for medicinal agents, a transdermal absorption promoting agent, a flavor, a colorant and the like, as an optional ingredient. Excipient
The excipient for use in the present invention is exemplified by silicon compounds such as anhydrous silicic acid, light anhydrous silicic acid and hydrous silicic acid, cellulose derivatives such as ethyl cellulose, methyl cellulose, hydroxypropyl cellulose and hydroxypropylmethyl cellulose, water soluble polymers such as polyvinyl alcohol, aluminum compounds such as dry aluminum hydroxide gel and hydrous aluminum silicate, kaolin, titanium oxide, and the like.

Anti-oxidizing Agent

The anti-oxidizing agent for use in the present invention is exemplified by dibutylhydroxytoluene, ascorbic acid, tocopherol, tocopherol ester derivatives, butylhydroxyanisole, 2-mercaptobenzimidazole, and the like.

Solubilizer for Drugs, Transdermal Absorption Promoting Agent

The solubilizer and transdermal absorption promoting agent for the drug for use in the present invention may be exemplified by polyhydric alcohols such as polyethylene glycol (average molecular weight: 200 to 30,000), glycerin, ethylene glycol and diethylene glycol, fatty acids such as oleic acid, citric acid and isostearic acid, fatty acid esters such as isopropyl myristate, isopropyl palmitate and diisopropyl adipate, fatty acid polyhydric alcohol esters such as caprylic acid monoglyceride, caprylic acid triglyceride and sorbitan fatty acid esters, terpenes such as menthol, menthol derivatives, mentha oil and limonene, polyvinyl alcohols, and the like. Of these, citric acid, isostearic acid, 1-menthol and the like are preferred. Thus, to blend a solubilizer that may lead to intense skin irritation such as crotamiton is not required since excellent drug solubility is provided according to the present invention. Therefore, enhancement of skin irritating properties can be suppressed.

Backing

The backing for use in the present invention is not particularly limited, and is exemplified by stretchable or non-stretchable woven or nonwoven fabrics of polyethylene, polypropylene etc., films of polyethylene, polypropylene, an ethylene vinyl acetate copolymer, vinyl chloride etc., or foamable backings of urethane, polyurethane etc., which may be used alone or as a laminated layer including multiple types of these.

Release Liner

The skin patch of the present invention has a backing and an adhesive layer laminated on one face of the backing, and is generally provided in the form in which a releasable liner is further included on an opposite face of the contact face of the adhesive layer and the backing.

As the releasable liner, a film of polyethylene, polypropylene, an ethylene vinyl acetate copolymer, vinyl chloride or the like, or a metal film deposited with aluminum vapor may be used. The liner subjected to a release-promoting treatment with silicon or the like on the surface may be also used. Furthermore, the liner may be also provided with a linear or curved notch for facilitating the release, and those including two or more liners layered in part, or having a turn-up are also acceptable.

EXAMPLES

Test Example 1 (Drug Solubility Test)

The amount of dissolution of loxoprofen sodium (LOX) with respect to 1 g of each additive was determined by visual observation. Liquid mixtures of each additive and N-methyl-2-pyrrolidone (NMP) were prepared in a mixing ratio shown in Table 1, and used in the test.

Table 1

| Additive | Mixing ratio | Amount of dissolved LOX (g) |
|---|---|---|
| NMP | - | 0 |
| Lactic acid | - | 0.25 |
| Triacetin | - | 0 |
| Oleyl alcohol | - | 0 |
| Polyethylene glycol 400 | - | 0.25 |
| Peppermint oil | - | 0 |
| 89% Phosphoric acid | - | 0.25 |
| Crotamiton | - | 0 |
| 10% Citric anhydride + NMP | 1:10 | 0.1 |
| 20% Citric anhydride + NMP | 1:5 | 0.1 |
| Phosphoric acid (89%)+NMP | 1:2 | 0 |
| Lactic acid + NMP | 1:2 | 1.5 |
| Isostearic acid + NMP | 1:1 | 0 |
| Myristic acid + NMP | 1:10 | 0 |
| Stearic acid + NMP | 1:1 | 0 |
| Behenic acid + NMP | 1:1 | 0 |
| Malic acid + NMP | 1:4 | 0 |
| Tartaric acid + NMP | 1:2 | 0 |
| Cetyl lactate + NMP | 1:9 | 0 |
| L-menthol + NMP | 1:1 | 0 |

As shown in Table 1, the amount of dissolution of loxoprofen sodium was markedly great only when lactic acid and N-methyl-2-pyrrolidone were used in combination. To the contrary, the amount of dissolution of loxoprofen sodium was significantly small when lactic acid or N-methyl-2-pyrrolidone was used alone, or other composition was employed.

Test Example 2 (Drug Stability Test)

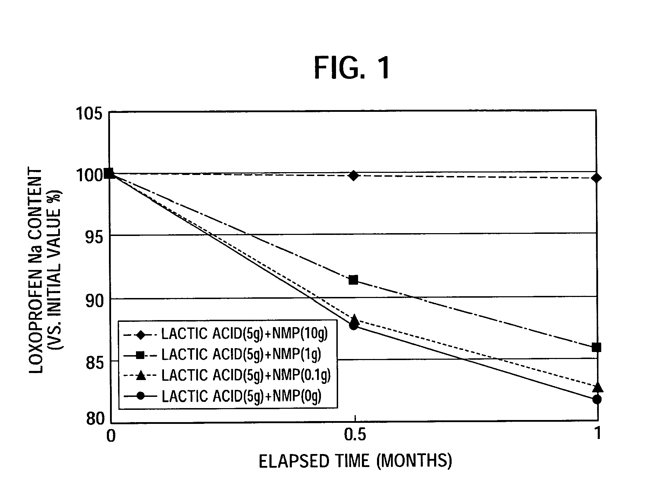

To each solvent was added 0.1 g of loxoprofen sodium, and the mixture was stirred at 25°C overnight. The stirred mixture was filtrated to obtain a filtrate for use as a sample solution, and this sample solution was left to stand at 60°C. Thereafter, the mass of loxoprofen sodium being present in each solvent system was measured with high performance liquid chromatography (HPLC) 0 months later (initial value), 0.5 months later, and 1.0 month later. The results are shown in Fig. 1 and Table 2 in terms of the ratio of each measurement with respect to the initial value.

Table 2

| Solvent system | Loxoprofen sodium content (%) (vs. initial value) | |
| --- | --- | --- |
| | 0.5 months later | 1.0 month later |
| Conc. glycerin 5 g | 87.2 | 82.4 |
| "Polysorbate 80 g" 5g | 83.5 | 69.1 |
| Peppermint oil 5g | 83.2 | 77.7 |
| Lactic acid 5 g | 83.1 | 79.9 |
| Lactic acid 5 g + NMP0.1g | 88.2 | 82.7 |
| Lactic acid 5 g + NMP1.0g | 91.3 | 85.8 |
| Lactic acid 5 g + NMP10.0g | 99.8 | 99.4 |

Test Example 3 (Drug Stability Test)

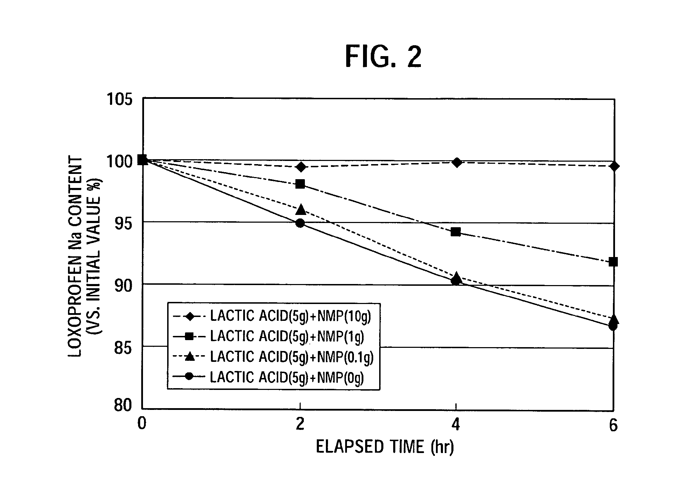

To each solvent was added 0.1 g of loxoprofen sodium, and the mixture was stirred at 25°C overnight. The stirred mixture was filtrated to obtain a filtrate for use as a sample solution. This sample solution was subjected to an autoclaving treatment at 120°C, and the mass of loxoprofen sodium being present in each solvent system was measured at time 0 (initial value), 2 hrs later, 4 hrs later, and 6 hrs later. The results are shown in Fig. 2 and Table 3 in terms of the ratio of each measurement with respect to the initial value. The measurement procedure was similar to Test Example 2.

Table 3

| Solvent system | Loxoprofen sodium content (%) (vs. initial value) | | |
| --- | --- | --- | --- |
| | 2 hrs | 4 hrs | 6 hrs |
| Lactic acid 5 g | 94.8 | 90.3 | 86.8 |
| Lactic acid 5 g + NMP0.1g | 96.0 | 90.7 | 87.4 |
| Lactic acid 5 g + NMP1.0g | 98.0 | 94.2 | 91.9 |
| Lactic acid 5 g + NMP10.0g | 99.4 | 99.8 | 99.6 |

As shown in Fig. 1 and Table 2, time dependent lowering of the content of loxoprofen sodium that is a component of a drug and has a carboxylic acid group, was greatly exhibited in the solvent system constituted with conc. glycerin, "polysorbate 80", mentha oil or lactic acid, each being a compound having a hydroxyl group. However, as shown in Tables 2 to 3 and Figs. 1 to 2, in the solvent system including lactic acid and N-methyl-2-pyrrolidone, time dependent lowering of the content of loxoprofen sodium was markedly suppressed. Therefore, is was suggested that use of the drug including a compound having a carboxylic acid group, the auxiliary ingredient including a compound having a hydroxyl group, and N-methyl-2-pyrrolidone in combination can improve the stability of the drug.

Test Example 4 (Drug Stability Test)

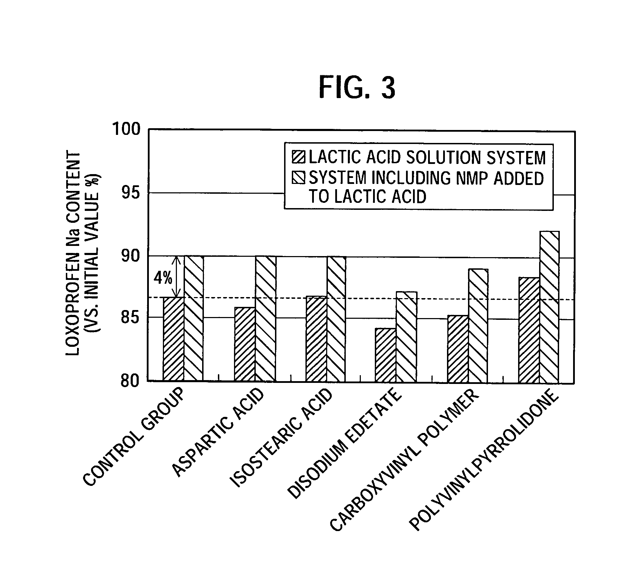

By a similar procedure to Test Example 3 except that 1 g of an additive (aspartic acid, isostearic acid, disodium edetate, a carboxyvinyl polymer or polyvinylpyrrolidone) was added to a mixed solution of 0.1 g of loxoprofen and 5 g of lactic acid, the mass of loxoprofen sodium being present in each solvent system was measured.
In addition, by a similar procedure to Test Example 3 except that 1 g of N-methyl-2-pyrrolidone, and 1 g of an additive (aspartic acid, isostearic acid, disodium edetate, the carboxyvinyl polymer or polyvinylpyrrolidone) were further mixed, the mass of loxoprofen sodium being present in each solvent system was measured. The results are shown in Fig. 3 in terms of the ratio of each measurement with respect to the initial value.

As shown in Fig. 3, the content of LOX found when the additive having a carboxylic acid group (aspartic acid, isostearic acid, disodium edetate and the carboxyvinyl polymer) was added was almost a similar level to the LOX content in the control group without the additive irrespective of the presence of NMP. Accordingly, it is considered that the stabilization effect of the drug in these solvent systems would be an effect resulting from NMP alone. Moreover, since NMP stabilized the drug when used in combination with any of the additives, excellent versatility of NMP was revealed.

In addition, favorable stability of LOX was achieved also when a compound having a carboxylic acid group other than those described above coexisted. For example, favorable stability of LOX was achieved also in a system in which NMP was added to LOX, lactic acid and adipic acid, or a system in which NMP was added to LOX, lactic acid and benzoic acid.

On the other hand, the content of LOX when polyvinylpyrrolidone was added increased to a level higher than the content of LOX in the control group irrespective of the presence of NMP. Accordingly, it was verified that the pyrrolidone compound such as polyvinylpyrrolidone exhibited a stabilization effect of the drug even though it was used alone, and also that use in combination with N-methyl-2-pyrrolidone can improve the stability of the drug synergistically.

Test Example 5 (Drug Stability Test)

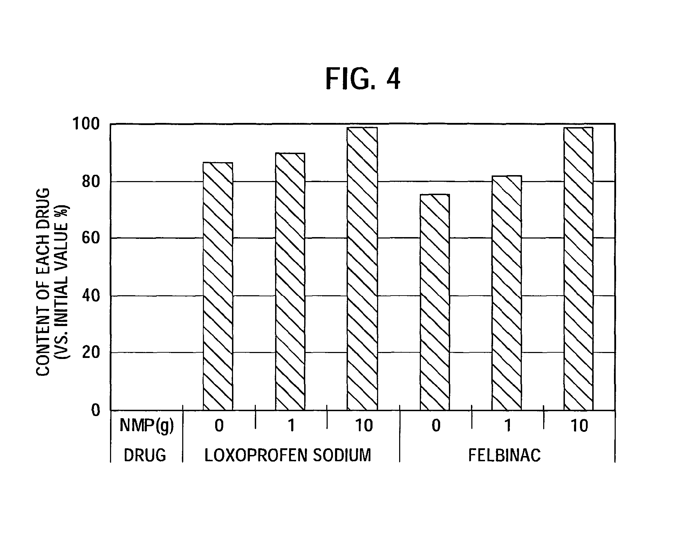

By a similar procedure to Test Example 3 except that either 1 g or 10 g of N-methyl-2-pyrrolidone was further mixed with a mixed solution of 0.1 g of loxoprofen sodium or felbinac and 5 g of lactic acid, the mass of the drug being present in each solvent system was measured. The results are shown in Fig. 4.

As shown in Fig. 4, stability of both loxoprofen sodium and felbinac was significantly improved by adding lactic acid and NMP. Accordingly, it was verified that the pyrrolidone compound can improve stability of a variety of drugs having a carboxylic acid group, not limited to loxoprofen sodium. Test Example 6 (Drug Stability Test)

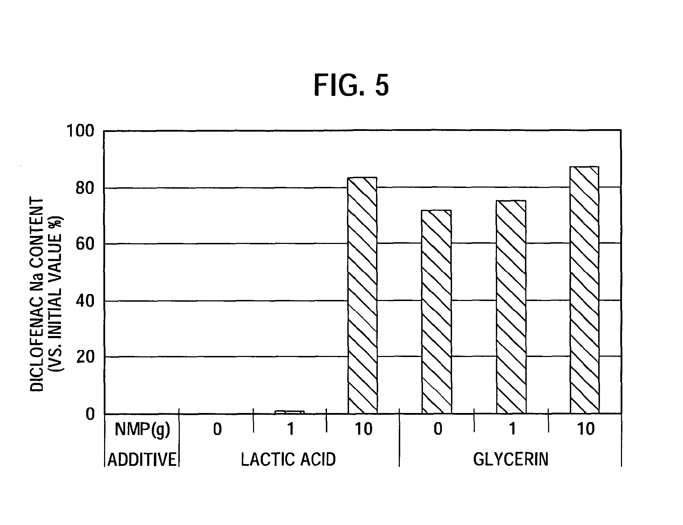

By a similar procedure to Test Example 5 except that a mixed solution of 0.1 g of diclofenac sodium and 5 g of lactic acid or glycerin was used, the mass of the drug being present in each solvent system was measured. The results are shown in Fig. 5.

As shown in Fig. 5, reduction of stability of diclofenac sodium caused by blending lactic acid was greatly compensated by adding NMP, whereby capability of improving stability of diclofenac sodium was confirmed. Moreover, addition of NMP also compensated the reduction of stability of diclofenac sodium caused by blending glycerin, whereby the capability of improving the stability of diclofenac sodium was also confirmed. Test Example 7 (Drug Stability Test)

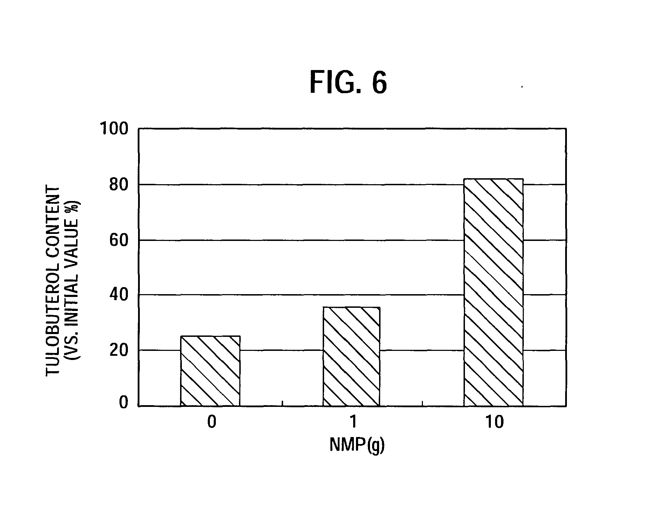

By a similar procedure to Test Example 5 except that a mixed solution of 0.1 g of tulobuterol and 5 g of acetic acid was used, the mass of the drug being present in each solvent system was measured. The results are shown in Fig. 6.

As shown in Fig. 6, it was verified that addition of acetic acid and NMP greatly improves the stability of tulobuterol. In view of all the results in the foregoing taking into consideration that tulobuterol has a hydroxyl group and acetic acid has a carboxylic acid group, it was ascertained that the stability of a drug can be improved by adding a pyrrolidone compound to a drug and an auxiliary ingredient, one of the drug and the auxiliary ingredient including a compound having a carboxylic acid group and the other of the drug and the auxiliary ingredient including a compound having a hydroxyl group.

The results are presumed to be due to suppression of the esterification reaction of the hydroxyl group and carboxylic acid group present in respective compounds included in the drug and the auxiliary ingredient, i.e., suppression of esterification of the drug, by the pyrrolidone compound such as N-methyl-2-pyrrolidone and polyvinylpyrrolidone.

Next, skin patches were produced, and the effects of use of the pyrrolidone compound and lactic acid in combination were verified by evaluation with each test. The present invention is explained below in more detail by way of Examples and Comparative Examples, but the present invention is not any way limited to these Examples.

Example 1

Liquid paraffin, Pinecrystal KE-100 (rosin ester derivative; manufactured by Arakawa Chemical Industries, Ltd.), dibutylhydroxytoluene and a hydrogenated oil (K-3 Wax 200; manufactured by Kawaken Fine Chemicals Co., Ltd.) were charged in a Henschel mixer (registered trademark), and the mixture was stirred to permit mixing. To this mixture was added a styrene-isoprene-styrene block copolymer (D-KX401CS; Kraton JSR Elastomers K.K.), and further 1-menthol, N-methyl-2-pyrrolidone, lactic acid and loxoprofen sodium were added thereto. The mixture was then stirred to permit mixing, whereby a pharmaceutical composition for external application was produced. Thus produced pharmaceutical composition for external application was stretched on a backing (fabric knitted with polyester, mass per unit area: 100 g/m$^2$) to form an adhesive layer, and a release liner film was laminated thereon to form a skin patch. The content of each ingredient was as shown in Table 4.

Example 2

A skin patch was obtained in a similar manner to Example 1 except that a hydrogenated oil was further added. The content of each ingredient was as shown in Table 4.

Comparative Example 1

A skin patch was obtained in a similar manner to Example 1 except that NMP was not included. The content of each ingredient was as shown in Table 4.

Comparative Example 2

A skin patch was obtained in a similar manner to Example 1 except that lactic acid was not included. The content of each ingredient was as shown in Table 4.

Comparative Example 3

A skin patch was obtained in a similar manner to Example 1 except that NMP and lactic acid were not included, and that triethanolamine (TEA) was included. The content of each ingredient was as shown in Table 4.

Comparative Example 4

A skin patch was obtained in a similar manner to Example 1 except that NMP and lactic acid were not included, and that phosphoric acid (89% phosphoric acid) was included. The content of each ingredient was as shown in Table 4.

With respect to Examples 1 to 2 and Comparative Examples 1 to 4, evaluation on releaseability and transdermal absorptivity of the medicinal agent was made. In addition, the presence of crystals of the drug in the skin patch was observed in order to confirm the solubility. The results are shown in Table 4.

Table 4

|  | Example 1 Lactic acid + NMP | Example 2 Lactic acid + NMP + Hydrogenated oil | Comparative Example 1 Lactic acid | Comparative Example 2 NMP | Comparative Example 3 TEA-based | Comparative Example 4 Phosphoric acid |
|---|---|---|---|---|---|---|
| LOX | 5 | 5 | 5 | 5 | 5 | 5 |
| SIS | 25 | 25 | 25 | 25 | 25 | 25 |
| LP | 37 | 35 | 39 | 37 | 37 | 40 |
| KE-100 | 25 | 25 | 25 | 25 | 25 | 25 |
| L-MEN | 1.5 | 1.5 | 1.5 | 1.5 | 1.5 | 1.5 |
| TEA | - | - | - | - | 4 | - |
| Lactic acid | 2 | 2 | 2 | - | - | - |
| Phosphoric acid | - | - | - | - | - | 1 |
| NMP | 4 | 4 | - | 4 | - | - |
| Hydrogenated oil | - | 2 | 2 | 2 | 2 | 2 |
| BHT | 0.50 | 0.50 | 0.50 | 0.50 | 0.50 | 0.50 |
| Presence of crystals | Absent | Absent | Absent | Present | Absent | Present |

(continued)

| | Example 1 Lactic acid + NMP | Example 2 Lactic acid + NMP + Hydrogenated oil | Comparative Example 1 Lactic acid | Comparative Example 2 NMP | Comparative Example 3 TEA-based | Comparative Example 4 Phosphoric acid |
|---|---|---|---|---|---|---|
| | | | | | | (unit: % by mass) |

LOX: loxoprofen sodium
SIS: styrene-isoprene-styrene block copolymer
LP: liquid paraffin
KE: KE-100 (rosin ester derivative)
L-MEN: 1-menthol
TEA: triethanolamine
NMP: N-methyl-2-pyrrolidone
BHT: dibutylhydroxytoluene

As shown in Table 4, crystals of the drug were observed in the skin patches of Comparative Example 2 and 4, while crystals of the drug were not observed in the skin patches of Examples 1 to 2. Therefore, the skin patches of Examples 1 to 2 were verified to have excellent drug solubility, suggesting that they can reduce skin irritation.

Test Example 4 (Release Test)

The test skin patches (Examples 1 to 2, and Comparative Examples 1 to 4) were applied onto skin extirpated from Yucatan Micropig, and left to stand under at a temperature of 32°C and a humidity of 60%. Each skin patch was removed 2 hrs, 8 hrs and 24 hrs later, and the remaining loxoprofen sodium in the test skin patch was extracted and quantitatively determined. The amount of the drug released from the skin patch and transferred to the skin was calculated from the amount of remaining drug based on the following formula, whereby evaluation was made on the releaseability from the skin patch. The results are shown in Table 5.

$$[((\text{Amount of drug before application}) - (\text{Amount of remaining drug}))/ \text{Amount of drug before application}] \times 100 =$$
$$\text{Proportion of drug release (\% by mass)}$$

Table 5

| | Release of loxoprofen sodium (% by mass) | | |
|---|---|---|---|
| Elapsed time | 2 hours later | 8 hours later | 24 hours later |
| Example 1 Lactic acid + NMP | 8.82 | 19.06 | 42.6 |
| Example 2 Lactic acid+NMP + Hydrogenated oil | 8.84 | 19.16 | 93.0 |
| Comparative Example 1 Lactic acid | 8.46 | 19.31 | 30.3 |
| Comparative Example 2 NMP | 1.45 | 1.74 | 5.3 |
| Comparative Example 3 TEA-based | 0.81 | 2.40 | 2.50 |
| Comparative Example 4 Phosphoric acid | 3.43 | 10.55 | 27.2 |

As shown in Table 5, the skin patches of Examples 1 to 2 released the drug in a larger amount than the skin patches of Comparative Examples 1 to 4 within predetermined time periods. Therefore, it was ascertained that the skin patches of Examples 1 to 2 exhibited extremely favorable releaseability of the medicinal agent.

Test Example 5 (Drug Transdermal Absorptivity Test)

Next, using the skin extirpated from a human, the skin patches of Examples 1 to 2 and Comparative Example 2 were subjected to a cutaneous permeability test of the drug. First, an isotonic phosphoric acid buffer (pH: 7.4) was applied on a dermis side (receiver side) of skin, which had been extirpated from a human, attached to a vertical diffusion cell, and each skin patch was applied on the stratum corneum side (donor side). Subsequently, after collecting receiver solution in a certain amount at each time point, the same amount of an isotonic phosphoric acid buffer was added thereto. Accordingly, the concentration of the drug in the collected receiver solution was measured by HPLC. The accumulated amount of the permeated drug was determined from the measurements. The steady-state permeation velocity (flux) was determined from this accumulated amount of the permeated drug, and the slope of the time curve, while the lag time defined by time section of the linear area was further determined. The results are shown in Table 6.

Table 6

| | | | Example 1 | Example 2 | Comparative Example 2 |
|---|---|---|---|---|---|
| Accumulated amount of permeated drug ($\mu$g/cm$^2$) | Elapsed time (hours) | 2 | 2.62 | 2.62 | 0.00 |
| | | 4 | 14.35 | 14.36 | 0.76 |
| | | 6 | 30.10 | 30.12 | 1.68 |
| | | 10 | 65.55 | 65.55 | 4.01 |
| | | 24 | 156.59 | 156.60 | 11.71 |
| | | 32 | 212.42 | 212.54 | 16.92 |
| | | 48 | 292.65 | 292.75 | 25.97 |
| flux($\mu$g/cm$^2$/hr) | | | 8.8 | 8.8 | 0.67 |
| Lag time(hours) | | | 3.18 | 3.18 | 6. 99 |

As shown in Table 6, the skin patches of Examples 1 to 2 could result in transdermal absorption of the drug in a larger amount than the skin patch of Comparative Example 2 within a short period of time. Therefore, it was ascertained that the skin patches of Examples 1 to 2 exhibited extremely favorable transdermal absorptivity of the drug.

Examples 3 to 4

Skin patches were obtained in a similar manner to Example 1 except that the ratio of addition of each ingredient was altered. The amount of each ingredient added was as shown in Table 7.

Table 7

| | Example 3 | Example 4 |
|---|---|---|
| SIS | 25 | |
| KE | 25 | |
| Hydrogenated oil | 0 | 2 |
| BHT | 0.5 | |
| LOX | 3.33 | |
| NMP | 2.67 | |
| Lactic acid | 1.33 | |
| L-MEN | 1.5 | |
| LP | 40.67 | 38.67 |

(continued)

|  | Example 3 | Example 4 |
|---|---|---|
| | (unit: % by mass) | |
| LOX: loxoprofen sodium<br>SIS: styrene-isoprene-styrene block copolymer<br>LP: liquid paraffin<br>KE: KE-100 (rosin ester derivative)<br>L-MEN: 1-menthol<br>NMP: N-methyl-2-pyrrolidone<br>BHT: dibutylhydroxytoluene | | |

Test Example 6 (Drug Stability Test)

Using the skin patches of Examples 3 to 4, a test on stability of loxoprofen sodium (LOX) was carried out. First, a fragment having a surface area of 10 cm$^2$ was cut away from each skin patch and the fragment was left to stand at 40°C or 60°C for 1.0 month. Thereafter, the mass of the test fragment was measured precisely. Then, the release liner film was peeled off, and the patch fragment was placed into a 50-mL centrifugal tube. Thereto was added a mixture of tetrahydrofuran and methanol, and shaken for 30 min, whereby the drug was extracted. The extract was subjected to high speed centrifugal separation, followed by filtration. The filtrate was used as a sample solution to determine the mass of the drug by HPLC.

In addition, the mass of the drug determined by a similar procedure in initiating the experiment (immediately after producing the skin patch) was defined as an initial value, and the LOC content ratio (%) was derived by calculating the proportion of the measured amount of the drug with respect to this initial value. The results are shown in Table 8.

Table 8

| Standing temperature | | 40°C | | 60°C | |
|---|---|---|---|---|---|
| | | Example 3 | Example 4 | Example 3 | Example 4 |
| LOX content ratio (%) (vs. initial LOX value) | | 94.3 | 98.5 | 89.1 | 93.2 |

As shown in Table 8, in any of the skin patches of Examples 3 to 4, time-dependent decrease of loxoprofen sodium was favorably suppressed. Among these, time-dependent decrease of loxoprofen sodium in the skin patch of Example 4 was extremely favorably suppressed. From these results, it was verified that addition of a hydrogenated oil to a solvent system including lactic acid and N-methyl-2-pyrrolidone can further improve the stability of the drug.

Examples 5 to 9

Skin patches were obtained in a similar manner to Example 1 except that: diclofenac sodium was added in place of loxoprofen sodium; lactic acid was not added; and that proportion of each ingredient added was altered. The amount of each ingredient added was as shown in Table 9.

Table 9

| | Example 5 | Example 6 | Example 7 | Example 8 | Example 9 |
|---|---|---|---|---|---|
| SIS | 27.5 | | | | |
| KE | 25.0 | | | | |
| Hydrogenated oil | 1.0 | | | 2.0 | |
| BHT | 0.5 | | | | |
| L-MEN | 1.5 | | | | |
| NMP | 9.0 | 3.0 | 2.0 | 4.0 | 3.0 |
| Citric acid | 0.4 | | | | |

(continued)

|  | Example 5 | Example 6 | Example 7 | Example 8 | Example 9 |
|---|---|---|---|---|---|
| DFNa | 1.0 | | | | |
| LP | 39.1 | 40.1 | 41.1 | 38.1 | 39.1 |
| | | | | | (unit: % by mass) |

DFNa: diclofenac sodium
SIS: styrene-isoprene-styrene block copolymer
LP: liquid paraffin
KE: KE-100 (rosin ester derivative)
L-MEN: 1-menthol
NMP: N-methyl-2-pyrrolidone
BHT: dibutylhydroxytoluene

Test Example 7 (Drug Stability Test)

Using the skin patches of Examples 5 to 9, stability of diclofenac sodium (DFNa) (suppressive property of diclofenac menthol ester (DME) production) was tested. First, after each skin patch was left to stand at 60°C for 1.5 months, the mass was measured precisely. Then, the release liner film was peeled off, and the patch fragment was placed into a 100-mL centrifugal tube. Thereto was added a mixture of ethyl acetate, hexane and methanol, and shaken for 30 min, whereby the drug and the like were extracted. The extract was subjected to high speed centrifugal separation, followed by filtration. The filtrate was used as a sample solution to determine the mass of the drug by HPLC.

In addition, the mass of the drug determined by a similar procedure in initiating the experiment (immediately after producing the skin patch) was defined as an initial value, and the rate of diclofenac menthol ester production (%) was derived using the following formula with the initial value as a control. The results are shown in Table 10.

```
Rate of DME production (%)

= Mass of DME in skin patch (%)/ Initial value of DFNa mass in skin patch (%) x 100
```

Table 10

| | Example 5 | Example 6 | Example 7 | Example 8 | Example 9 |
|---|---|---|---|---|---|
| Rate of DME production (%) | 3.34 | 9.62 | 5.81 | 3.22 | 4.89 |

As shown in Table 10, the skin patches of Examples 5 to 9 kept the amount of production of the diclofenac sodium menthol ester at low levels. Thus, it was verified that the addition of N-methyl-2-pyrrolidone to the adhesive layer including diclofenac sodium that is a component of a drug and has a carboxylic acid group, and 1-menthol that is a component of an auxiliary ingredient and has a hydroxyl group can improve stability of diclofenac sodium. Moreover, it was also verified that the stability of diclofenac sodium can be additionally improved by further adding a hydrogenated oil.

From the foregoing results, it was proven that a skin patch which exhibits superior solubility, releaseability and stability of a drug, and which is superior in transdermal absorptivity can be obtained by blending a pyrrolidone compound such as N-methyl-2-pyrrolidone into an adhesive layer including the drug and an auxiliary ingredient, one thereof including a compound having a carboxylic acid group and the other thereof including a compound having a hydroxyl group.

**Claims**

1.　A pharmaceutical composition for external application to skin,
the composition comprising a drug, an auxiliary ingredient, and a pyrrolidone compound, wherein
one of the drug and the auxiliary ingredient comprises a compound including a carboxylic acid group, and an other of the drug and the auxiliary ingredient comprising a compound including a hydroxyl group.

2. The composition according to claim 1, wherein the pyrrolidone compound is at least one selected from the group consisting of N-methyl-2-pyrrolidone, 2-pyrrolidone, 5-methyl-2-pyrrolidone, 1,5-dimethyl-2-pyrrolidone, 1-ethyl-2-pyrrolidone, 2-pyrrolidone-5-carboxylic acid, N-(2-hydroxyethyl)pyrrolidone, N-octyl-2-pyrrolidone, polyvinylpyrrolidone, N-vinyl-2-pyrrolidone, and methylvinylpyrrolidone.

3. The composition according to claim 2, wherein the pyrrolidone compound is N-methyl-2-pyrrolidone.

4. The composition according to any one of claims 1 to 3, further comprising a hydrogenated oil.

5. The composition according to any one of claims 1 to 4, wherein the compound included in the drug is at least one selected from the group consisting of loxoprofen, diclofenac, felbinac, ketoprofen, indometacin, tulobuterol, and a salt thereof.

6. The composition according to any one of claims 1 to 5, wherein the compound included in the auxiliary ingredient is at least one selected from the group consisting of a hydroxy acid, a menthols, and a polyhydric alcohol.

7. The composition according to claim 6, wherein the hydroxy acid is at least one selected from the group consisting of lactic acid, tartaric acid, and citric acid.

8. A skin patch comprising a backing, and an adhesive layer laminated on the backing, wherein the adhesive layer comprises the composition according to any one of claims 1 to 7.

9. The skin patch according to claim 8, wherein the pyrrolidone compound is included in an amount of no less than 0.01% by mass and no greater than 20% by mass based on the mass of the entire adhesive layer.

10. The skin patch according to claim 8 or 9, wherein the adhesive layer comprises a styrene-isoprene-styrene block copolymer.

11. A skin patch comprising a backing, and an adhesive layer laminated on the backing, wherein the adhesive layer comprises a styrene-isoprene-styrene block copolymer, a tackifier, a plasticizer, diclofenac sodium, 1-menthol, N-methyl-2-pyrrolidone, a hydrogenated oil, and at least one of citric acid and lactic acid.

12. A method for suppressing esterification of respective compounds included in a drug and an auxiliary ingredient included in a pharmaceutical composition for external application to skin, wherein one of the drug and the auxiliary ingredient comprises a compound including a carboxylic acid group, and an other of the drug and the auxiliary ingredient comprises a compound including a hydroxyl group.

13. An esterification suppressing agent for use in a pharmaceutical composition for external application to skin, the composition comprising a drug and an auxiliary ingredient, wherein one of the drug and the auxiliary ingredient comprises a compound including a carboxylic acid group, and an other of the drug and the auxiliary ingredient comprises a compound including a hydroxyl group, and wherein the agent comprises a pyrrolidone compound in an effective amount for suppressing esterification of the drug and the auxiliary ingredient.

# FIG. 1

# FIG. 2

# FIG. 3

# FIG. 4

FIG. 5

# FIG. 6

<table>
<tr><td colspan="2" align="center">**INTERNATIONAL SEARCH REPORT**</td><td colspan="2">International application No.<br>PCT/JP2007/073616</td></tr>
</table>

A. CLASSIFICATION OF SUBJECT MATTER
*A61K9/70*(2006.01)i, *A61K31/137*(2006.01)i, *A61K31/192*(2006.01)i, *A61K31/195* (2006.01)i, *A61K31/405*(2006.01)i, *A61K47/10*(2006.01)i, *A61K47/12*(2006.01)i, *A61K47/22*(2006.01)i, *A61K47/32*(2006.01)i, *A61K47/44*(2006.01)i

B. FIELDS SEARCHED

Minimum documentation searched (classification system followed by classification symbols)
A61K9/70, A61K31/137, A61K31/192, A61K31/195, A61K31/405, A61K47/10, A61K47/12, A61K47/22, A61K47/32, A61K47/44

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
JMEDPlus(JDream2), JST7580(JDream2), JSTPlus(JDream2)

C. DOCUMENTS CONSIDERED TO BE RELEVANT

| Category* | Citation of document, with indication, where appropriate, of the relevant passages | Relevant to claim No. |
|---|---|---|
| X | JP 2002-020274 A (San-A Seiyaku Kabushiki Kaisha), 23 January, 2002 (23.01.02), Claims 1 to 12; Par. Nos. [0012], [0038]; examples (Family: none) | 1-13 |
| X | JP 08-113537 A (Saitama Daiichi Pharmaceutical Co., Ltd.), 07 May, 1996 (07.05.96), Claims 1 to 3; Par. Nos. [0006] to [0028]; table 1 & JP 3382032 B2 | 1-13 |

☒ Further documents are listed in the continuation of Box C.  ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>16 January, 2008 (16.01.08) | Date of mailing of the international search report<br>29 January, 2008 (29.01.08) |
|---|---|
| Name and mailing address of the ISA/<br>Japanese Patent Office | Authorized officer |
| Facsimile No. | Telephone No. |

Form PCT/ISA/210 (second sheet) (April 2007)

| | INTERNATIONAL SEARCH REPORT | International application No. |
|---|---|---|
| | | PCT/JP2007/073616 |

C (Continuation).   DOCUMENTS CONSIDERED TO BE RELEVANT

| Category* | Citation of document, with indication, where appropriate, of the relevant passages | Relevant to claim No. |
|---|---|---|
| X | JP 11-222443 A  (Saitama Daiichi Pharmaceutical Co., Ltd.), 17 August, 1999 (17.08.99), Claims 1 to 10; Par. Nos. [0006] to [0025], [0050] (Family: none) | 1-13 |
| X<br><br>A | Minako YOSHIDA, et al., "Acrylic Acid-kei Kobunshi o Mochiita Pap-zai kara no Ketoprofen Hifu Toka", The Pharmaceutical Society of Japan Dai 122 Nenkai Koen Yoshishu, 05 March, 2002 (05.03.02), Vol.122, No.4, page 55, 26[P] I-403 Ran [Hoho] Oyobi [Kekka·Kosatsu] Ran Sansho/ ISSN:0918-9823 | 1-3,5,6,<br>8-10,12,13<br>4,7,11 |
| X<br><br>A | JP 2002-338462 A  (Tokuhon Corp., SS Pharmaceutical Co., Ltd.), 27 November, 2002 (27.11.02), Claims 1 to 5; Par. Nos. [0005], [0014] & WO 2002/094257 A1      & EP 1312360 A1 & KR 2003016263 A       & US 2003/175331 A1 & CN 1462187 A           & AU 2002258212 A1 & CN 1248685 C | 1-3,5-10,12,<br>13<br>4,11 |
| X<br><br>A | JP 2001-064205 A  (Daiichi Pharmaceutical Co., Ltd., Saitama Daiichi Pharmaceutical Co., Ltd.), 13 March, 2001 (13.03.01), Claims 1 to 31; Par. Nos. [0041] to [0059] (Family: none) | 1-3,5-10,12,<br>13<br>4,11 |
| A | JP 2004-083462 A  (Yutoku Pharmaceutical Ind. Co., Ltd.), 18 March, 2004 (18.03.04), Par. No. [0009] & JP 3668728 B2 | 1-13 |
| P,X<br>P,A | JP 2007-008927 A  (Saitama Daiichi Pharmaceutical Co., Ltd.), 18 January, 2007 (18.01.07), Claims 1 to 8; Par. Nos. [0010] to [0055] (Family: none) | 11<br>12,13 |

Form PCT/ISA/210 (continuation of second sheet) (April 2007)

| INTERNATIONAL SEARCH REPORT | International application No. |
|---|---|
| | PCT/JP2007/073616 |

<Subject of the search over the prior art documents>

In claims 1-11, the active ingredients are defined as "comprising a pharmacologically active ingredient, an auxiliary component, and a pyrrolidone compound, wherein one of the pharmacologically active ingredient and the auxiliary component has a carboxylate group in the molecule and the other has a hydroxy group in the molecule" (claim 1). Thus, the active ingredients are defined only by the presence of specific functional groups in the molecule, not by the structure. Similar expressions are also found in claims 12 and 13.

With regard to the active ingredients for the inventions of the above-mentioned claims, specific examples of the active ingredients are mentioned in Paragraph Nos. [0027]-[0038] in the description. However, there is found no explanation in the description as to what types of substances are included within the scopes of the above-defined active ingredients. Further, even though the common technical knowledge at the time of filing the present application is taken into the consideration, it does not appear that the scopes of the active ingredients can be specified. Therefore, claim 1 does not comply with the requirement of clearness under PCT Article 6, too. Among the above-specified active ingredients, those which are disclosed in the meaning within PCT Article 5 and supported in the meaning within PCT Article 6 are limited to loxoprofen sodium, diclofenac sodium, 1-menthol, lactic acid, N-methyl-2-pyrrolidone.

Such being the case, in this international search report, with respect to claims 1-13, the search was made only on the case where the active ingredients are those compounds specifically described in claims 2 and 5-7, with the statements of the description being taken into consideration.

Form PCT/ISA/210 (extra sheet) (April 2007)

**EP 2 116 234 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004043512 A
- JP HEI10120560 B
- JP HEI747535 B
- WO 2006048939 A